# EUROPÄISCHE PATENTSCHRIFT

(11) **EP 2 310 066 B1**
(45) Veröffentlichungstag und Bekanntmachung des Hinweises auf die Patenterteilung: **15.03.2017**
(21) Anmeldenummer: 09775747.0
(22) Anmeldetag: 14.07.2009
(51) Int. Cl.: A61M 1/00

(54) **FLUIDSAMMELBEHÄLTER**
FLUID-COLLECTING CONTAINER
RÉSERVOIR DE COLLECTE DE FLUIDE

(30) Priorität: 15.07.2008 CH 10982008
(43) Veröffentlichungstag der Anmeldung: 20.04.2011
(73) Patentinhaber: Medela Holding AG, 6340 Baar (CH)
(72) Erfinder: HOFMANN, Adrian, CH-6005 Luzern (CH); IMBODEN, David, 6340 Baar (CH)
(74) Vertreter: Clerc, Natalia
(86) Internationale Anmeldenummer: PCT/CH2009/000250
(87) Internationale Veröffentlichungsnummer: WO 2010/006458

(56) Entgegenhaltungen:
- EP-A- 0 144 105
- EP-A- 0 390 094
- DE-A1- 19 836 497
- JP-A- 2003 275 257
- US-A- 4 386 930
- US-A- 5 225 158
- US-A- 5 589 145

## Beschreibung

### Technisches Gebiet

Die Erfindung betrifft einen Fluidsammelbehälter zur Aufnahme eines abgesaugten Fluids gemäss Oberbegriff des Patentanspruchs 1.

### Stand der Technik

Zum Absaugen von Körperflüssigkeiten oder Sekreten aus Körperkavitäten oder Wunden werden im medizinischen Bereich, insbesondere in der Thoraxdrainage, stationäre oder mobile Absaugsysteme verwendet. Diese Systeme umfassen jeweils eine Saugquelle, beispielsweise eine Vakuumpumpe oder ein Zentralvakuum, und einen oder mehrere Drainagebehälter. Eine Vakuumleitung verbindet den Drainagebehälter mit der Saugquelle und eine Drainageleitung führt vom Drainagebehälter zum Patienten. Das angelegte Vakuum erzeugt einen Unterdruck im Behälter und die abzusaugende Flüssigkeit bzw. das abzusaugende Sekret wird vom Patienten in den Drainagebehälter abgesaugt und dort gesammelt. Der Drainagebehälter kann starr oder als flexibler Beutel ausgebildet sein. Der Beutel kann wiederum in einem starren, luftdicht verschliessbaren Aussenbehälter angeordnet sein.

Ein mit abgesaugter Körperflüssigkeit, insbesondere Blut, bzw. mit Sekret gefüllter Behälter muss fachgerecht entsorgt werden. Beim Transport und der Entsorgung des Behälters besteht jedoch die Gefahr, dass Flüssigkeit nach aussen gelangen kann, beispielsweise wenn der Behälter nicht genügend dicht verschlossen wurde. Um die Infektionsgefahr zu minimieren, ist es deshalb im Stand der Technik bekannt, die gesammelte Flüssigkeit bereits im Behälter zu verfestigen. Derartige Verfestigungsmittel sind beispielsweise in EP-A-0 839 539 und US 2007/0185366 offenbart.

Es sind verschiedene Vorrichtungen bekannt, um diese Verfestigungsmittel gezielt erst bei Gebrauch in den Behälter einzubringen. So offenbart beispielsweise EP-A-0 668 084 eine Drainagevorrichtung mit einem starren Aussenbehälter und einem flexiblen Innenbeutel, welcher an einem den Aussenbehälter verschliessenden Deckel angebracht ist. Im Deckel sind ein Vakuumanschluss zur Verbindung mit einer Saugquelle und ein Drainageanschluss zur Verbindung mit einem Patientenschlauch vorhanden. Am Drainageanschluss, zur Beutelinnenseite hin gerichtet, ist ein Behälter angeordnet, welcher ein Verfestigungs- und Desinfektionsmittel enthält. Der Behälter wird beim Anlegen des Vakuums automatisch geöffnet.

EP-A-1 642 603 beschreibt einen Drainagebeutel mit einem darin angeordneten Behälter für ein Gerinnungsmittel. Der Behälter ist wasserlöslich und löst sich bei Kontakt mit einer abgesaugten Körperflüssigkeit auf, so dass das Gerinnungsmittel in den Drainagebeutel freigegeben wird.

JP 5329471 zeigt einen Drainagebehälter mit einem Deckel, wobei im Deckel ein Sauganschluss und ein Vakuumanschluss vorhanden sind. Zusätzlich ist im Deckel eine mit einem Verfestigungsmittel gefüllte Kammer vorhanden. Das Verfestigungsmittel lässt sich mittels eines Stössels aus der Kammer in den Drainagebehälter befördern, um so die in den Behälter abgesaugte Körperflüssigkeit zu binden.

Auch in JP 7060231 ist ein Drainagebehälter mit Deckel und einer aufgesetzten Kammer gezeigt, wobei die Kammer wiederum ein Verfestigungsmittel beinhaltet. Ein Stöpsel verschliesst eine in den Drainagebehälter ragende Öffnung der Kammer. Dieser Stöpsel lässt sich über einen von aussen betätigbaren Hebel nach unten drücken, so dass die Öffnung freigegeben wird und das Verfestigungsmittel in den Drainagebehälter gelangen kann.

JP 7303875 zeigt ebenfalls eine auf einem Drainagebehälter aufgesetzte Kammer für ein Verdickungsmittel, welche über einen relativ kompliziert aufgebauten und von aussen manuell betätigbaren Mechanismus geöffnet werden kann.

US 5 225 158 zeigt einen Behälter zum Sammeln von flüssigen Abfallstoffen, die mittels eines Unterdrucks angesaugt werden. Der Behälter weist einen Deckel auf, an dessen Unterseite eine Kammer zur Lagerung einer Desinfektionsflüssigkeit angebracht ist. Auf der Unterseite dieser Kammer gibt es in der Kammerwand eine dünne Stelle. Durch Eindrücken eines Bedienelementes, das innerhalb der Kammerwand und auf der gegenüberliegenden Seite der dünnen Stelle angeordnet ist, wird ein Stössel in Richtung der dünnen Stelle bewegt. Bei vollständigem Eindrücken des Bedienelements durchstösst der angespitzte Stössel dabei die dünne Stelle und die Desinfektionsflüssigkeit gelangt aus der Kammer in den restlichen Behälterinnenraum hinein.

Diese bekannten Vorrichtungen sind relativ kompliziert aufgebaut und entsprechend teuer in der Herstellung. Die aussen aufgesetzten Kammern weisen zudem den Nachteil auf, dass sie bei der Lagerung und beim Transport des Behälters stören und auch leicht beschädigt werden können.

### Darstellung der Erfindung

Es ist deshalb eine Aufgabe der Erfindung, einen Fluidsammelbehälter mit einer Kammer für ein Verfestigungsmittel zu schaffen, welcher die oben genannten Nachteile behebt.

Diese Aufgabe löst ein Fluidsammelbehälter mit den Merkmalen des Patentanspruchs 1.

Der erfindungsgemässe Drainagebehälter zur Aufnahme eines abgesaugten Fluids weist eine geschlossene Kammer für ein Verfestigungsmittel auf, wobei sich die Kammer durch äussere Krafteinwirkung zu einem Innenraum des Fluidsammelbehälters hin öffnen lässt. Die Kammer ragt mindestens teilweise in den Innenraum des Fluidsammelbehälters hinein. Die Kammer ist behälteraussenseitig mit einer nach innen drückbaren äusseren Wand verschlossen, wobei sich die Kammer durch das Eindrücken der äusseren Wand zum Innenraum des Fluidsammelbehälters hin öffnet, wobei der Innenraum in diesem Bereich weiterhin nach aussen mit der äusseren Wand verschlossen ist. Der Fluidsammelbehälter weist ein erstes und ein zweites Gehäuseteil auf, welche gemeinsam den Innenraum des Fluidsammelbehälters bilden. Die äussere Wand ist einstückig mit einer sie umgebenden Wand des ersten Gehäuseteils ausgebildet.

Diese äussere eindrückbare Wand bildet somit eine dichte Barriere nach aussen sowohl vor wie auch nach dem Öffnen der Kammer, so dass in diesem Bereich keine Flüssigkeit und auch kein verdicktes Material vom Innenraum nach aussen gelangen kann. Auch die Kammer verbleibt in diesem Bereich vorzugsweise mit dieser äusseren Wand verschlossen.

Obwohl hier beschrieben ist, dass der Innenraum verschlossen ist, kann er seinen anderen Bereichen trotzdem Öffnungen nach aussen aufweisen, beispielsweise für die Vakuum- und Drainageanschlüsse. "Dieser Bereich" meint hier den Bereich, welcher durch die eindrückbare äussere Wand gebildet ist.

In einer ersten Ausführungsform lässt sich die äussere Wand zum Innenraum hin verschieben, wobei sie nach wie vor eine zumindest flüssigkeitsdichte Verbindung mit der restlichen Wandung des Drainagebehälters aufweist. Vorzugsweise bleibt sie jedoch an ihren Rändern lagestabil und lässt sich lediglich mit ihrem inneren Bereich nach innen bewegen. Vorzugsweise ist sie flexibel ausgestaltet.

In einer einfachen Ausführungsform wird durch das Eindrücken der äusseren Wand der Druck in der Kammer so erhöht, dass Schwachstellen in ihrer zum Innenraum des Behälters gerichteten Wandung aufgebrochen werden, wodurch das Verfestigungsmittel in den Innenraum gelangen kann. In einer bevorzugten Ausführungsform ist jedoch mindestens ein Übertragungsmittel vorhanden, welches die Bewegung der äusseren Wand auf mindestens eine innen liegende Wand der Kammer überträgt, um die Kammer zu öffnen. Vorzugsweise ist das Übertragungsmittel mindestens ein Stössel, welcher auf einen der äusseren Wand gegenüberliegenden mindestens teilweise entfernbaren Deckel der Kammer wirkt. Der Stössel ist vorzugsweise mit der äusseren Wand oder mit dem Deckel verbunden, vorzugsweise ist er einstückig mit der Wand bzw. dem Deckel ausgebildet. Vorzugsweise ist er zentrisch in der Kammer angeordnet. Die zum Öffnen benötigte Bewegung der äusseren Wand kann minimiert werden, wenn sich der Stössel fast annähernd oder genau über die gesamte Länge der Kammer erstreckt.

Der Stössel kann zentral angeordnet sein. Er kann jedoch auch peripher oder an einer anderen geeigneten Lage angeordnet sein. Anstelle eines stiftartigen Stössels lässt sich auch ein hohlzylinderförmiger Stössel oder ein Stössel mit einer anderen Grundform verwenden. Der Stössel kann hohl oder mit Material ausgefüllt sein. Es lassen sich auch mehrere Stössel einsetzen. Beispielsweise können alternativ bzw. zusätzlich zu einem zentralen Stössel mehrere Stössel gleichabständig und kreisförmig um eine Mittelachse der Kammer angeordnet sein. Ferner können die Stössel gleiche oder unterschiedliche Längen aufweisen.

Vorzugsweise ist die bewegbare Wand in einer Aussenwand des Sammelbehälters angeordnet, wobei sie vorzugsweise vertieft in ihr angeordnet ist. Ist die Kammer im Sammelbehälter versenkt, ist sie während des Transports und der Lagerung besser geschützt.

Da die Kammer integraler Bestandteil des Innenraums ist und da die eindrückbare äussere Wand einstückig mit der genannten sie umgebenden Wand des Flüssigkeitssammelbehälters verbunden ist, wird die Herstellung erleichtert. Zudem ist gewährleistet, dass der Innenraum bis auf Drainage- und Vakuumanschlüsse und allfällige Überdruckventile luft- und flüssigkeitsdicht ist. Diese Vorteile werden noch verstärkt, wenn die Kammer einen in den Innenraum des Fluidsammelbehälters ragenden Mantel aufweist, welcher einstückig mit der die Kammer umgebenden Wand des Fluidsammelbehälters ausgebildet ist. Dasselbe gilt, wenn der Fluidsammelbehälter vorzugsweise inkl. der Kammer aus Kunststoff gefertigt ist, beispielsweise aus Polypropylen (PP).

Die Kammer lässt sich in unterschiedlichen Formen ausbilden. Sie kann beispielsweise kugelförmig, eiförmig oder quaderförmig sein. Vorzugsweise ist sie jedoch zylinderförmig und die äussere Wand ist vorzugsweise kreisförmig ausgebildet.

Weitere vorteilhafte Ausführungsformen gehen aus den abhängigen Patentansprüchen hervor.

### Kurze Beschreibung der Zeichnungen

Im Folgenden wird der Erfindungsgegenstand anhand eines bevorzugten Ausführungsbeispiels, welches in den beiliegenden Zeichnungen dargestellt ist, erläutert. Es zeigen:
- Figur 1: eine Explosionsdarstellung des erfindungsgemässen Fluidsammelbehälters;
- Figur 2: eine perspektivische Darstellung des Fluidsammelbehälters gemäss Figur 1;
- Figur 3: eine perspektivische Darstellung des Innenraums eines ersten Teils des Fluidsammelbehälters;
- Figur 4: eine erste Seitenansicht des ersten Teils des Fluidsammelbehälters;
- Figur 5: einen Längsschnitt durch den Fluidsammelbehälter;
- Figur 6: eine zweite Seitenansicht des ersten Teils des Fluidsammelbehälters und
- Figur 7: einen Längsschnitt durch eine Kammer des Fluidsammelbehälters in einer vergrösserten Darstellung.

### Wege zur Ausführung der Erfindung

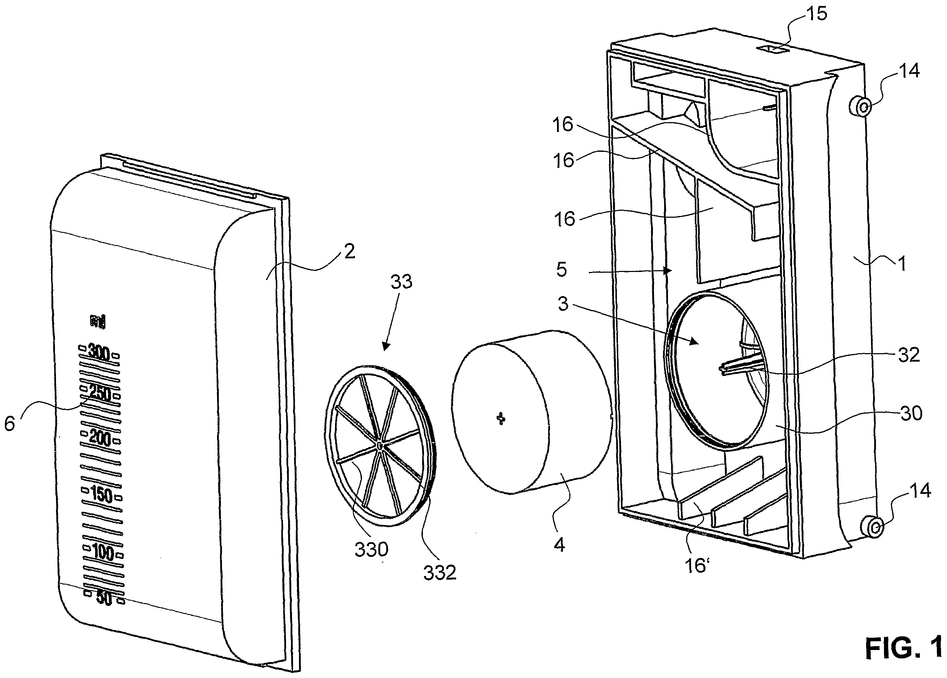

In Figur 1 ist ein erfindungsgemässer Drainage- oder Fluidsammelbehälter dargestellt. Er wird wie die eingangs beschriebenen Behälter im medizinaltechnischen Bereich zur Aufnahme von abgesaugten Körperflüssigkeiten, wie beispielsweise Wasser, Blut oder Fett, eingesetzt. Die Absaugung erfolgt insbesondere im operativen oder postoperativen Bereich oder während der Krankheitsbehandlung. Eine wichtige Anwendung ist die Thoraxdrainage.

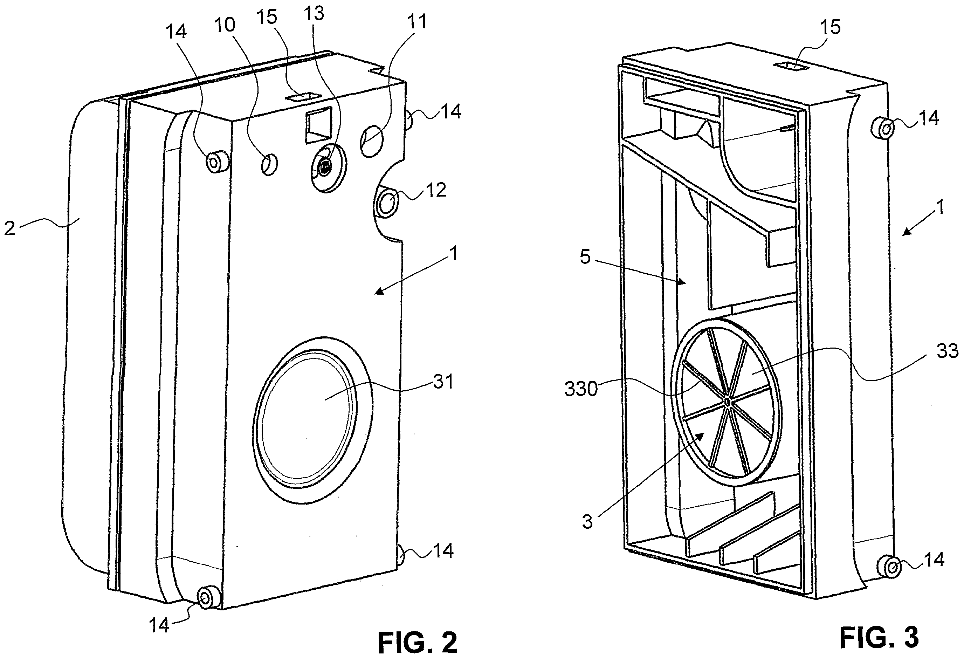

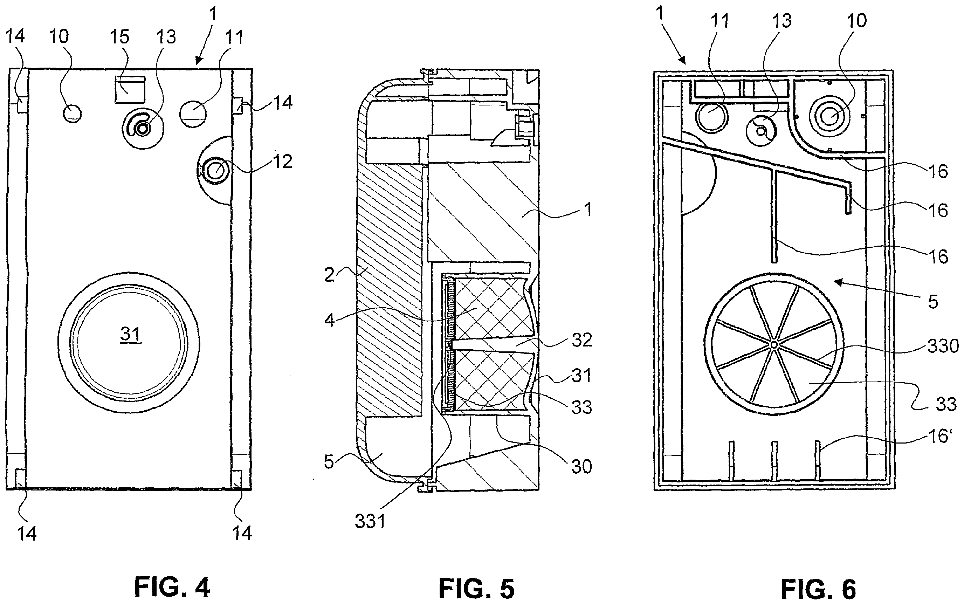

Der dargestellte Fluidsammelbehälter weist ein erstes Behälterteil 1 und ein zweites Behälterteil 2 auf. Die zwei Behälterteile 1, 2 sind flüssigkeits- und luftdicht miteinander verbunden. Vorzugsweise werden sie zusammen gesteckt und miteinander verklebt oder verschweisst. Die zwei Behälterteile 1, 2 bilden einen gemeinsamen Innenraum, die Sekretkammer 5. Im hier dargestellten Beispiel sind noch weitere Kammern vorhanden, welche durch Rippen 16 von der Sekretkammer getrennt, aber über Kanäle mit dieser verbunden sind. Des Weiteren können in der Sekretkammer 5 weitere Rippen 16' angeordnet sein, welche Schwappbewegungen der abgesaugten Flüssigkeit innerhalb der Sekretkammer vermindern. Dies ist insbesondere in den Figuren 3 und 6 gut erkennbar.

Der Fluidsammelbehälter weist ferner eine Kammer 3 für ein Verfestigungsmittel 4 auf. Diese Kammer 3 wird weiter unten im Text detaillierter beschrieben.

Der Fluidsammelbehälter, hier das zweite Gehäuseteil 2, weist vorzugsweise mindestens eine Füllstandskala 6 auf. Diese Füllstandskala 6 ist nichtlinear und berücksichtigt den durch die Kammer 3 eingenommenen Raum in der Sekretkammer 5.

In Figur 2 ist der Behälter im zusammengesetzten Zustand dargestellt. Er lässt sich mittels seiner seitlich vorstehenden Befestigungszapfen 14 an eine Saugquelle, insbesondere eine Vakuumpumpe, anhängen. Eine Ausnehmung 15 dient der Aufnahme einer Verriegelungstaste dieser Vakuumpumpe. Im ersten Gehäuseteil 1 sind Durchgangsöffnungen vorhanden, welche in das Innere des Behälters führen. Diese Durchgangsöffnungen bilden einen Vakuumanschluss 10 und einen Drainageanschluss 11. Der Vakuumanschluss dient zur Verbindung mit der Vakuumpumpe, entweder, wie in diesem Beispiel, über eine direkte interne Leitung oder über einen Vakuumschlauch. Der Drainageanschluss 11 dient zur Verbindung mit einem Drainageschlauch, welcher zur abzusaugenden Kavität des Patienten führt. Auch diese Verbindung kann je nach Art der Saugquelle direkt durch Einstecken des Drainageschlauchs in diesen Drainageanschluss 11 oder, wie in diesem Beispiel, über eine Verbindung mit dem Gehäuse der Vakuumpumpe erfolgen. Der Behälter kann vorzugsweise ein oder mehrere Überdruckventile 13 aufweisen, welche sich bei Überdruck in der Sekretkammer 5 öffnen.

Wie in Figur 4 dargestellt ist, kann das erste oder zweite Gehäuseteil 1, 2 ferner einen Verschlussteil 12, insbesondere einen Zapfen, zum Verschliessen des Drainageanschlusses aufweisen. Dieser lässt sich bei Bedarf vom Gehäuseteil entfernen.

Die zwei Gehäuseteile 1, 2 sind vorzugsweise aus Kunststoff, vorzugsweise PP, gefertigt. Vorzugsweise sind beide für sich einstückig hergestellt, wobei das entfernbare Verschlussteil 12 mit angespritzt ist.

Erfindungsgemäss ist nun im Innenraum des Behälters die geschlossene Kammer 3 angeordnet, welche mindestens teilweise, vorzugsweise ganz, in diesen Innenraum hineinragt. Die Kammer 3 ist in diesem Beispiel zylinderförmig gestaltet und weist einen hohlzylinderförmigen Mantel 30 auf, welcher einer Rückwand des ersten Behälterteils 1 in die Sekretkammer 5 hinein vorsteht. Eine erste Seite des Mantels 30 ist mit einer äusseren Wand 31 verschlossen. Eine zweite, gegenüberliegende Seite des Mantels 30 ist mit einem Deckel 33 verschlossen. An der äusseren Wand 31 ist zentrisch ein Stössel 32 angeordnet, welcher sich bis zum Deckel 33 erstreckt und vorzugsweise in einer Aufnahme 331 desselben angeordnet ist.

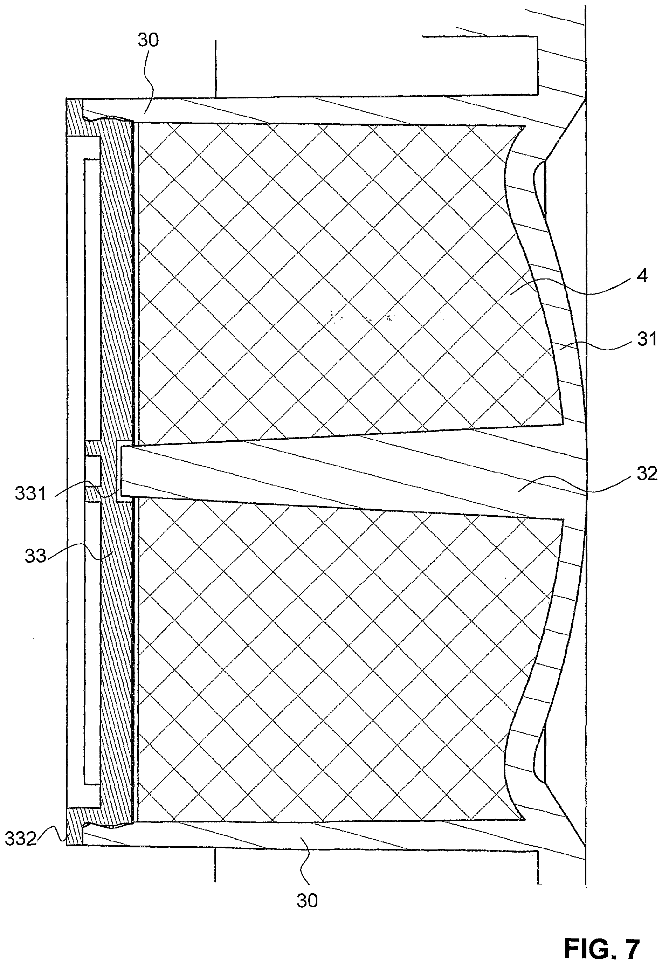

Der Mantel 30, die äussere Wand 31 und der Stössel 32 sind vorzugsweise einstückig mit der sie umgebenden Wand des ersten Gehäuseteils 1, vorzugsweise mit dem gesamten restlichen Gehäuseteil 1, gemeinsam einstückig ausgebildet. Dies ist insbesondere in den Figuren 5 und 7 gut erkennbar. Die äussere Wand 31 ist vorzugsweise flexibel oder zumindest weicher ausgebildet als der sie umgebende Bereich des restlichen Gehäuseteils 1. Vorzugsweise ist sie über eine scharnierartige oder zumindest verdünnte Verbindung mit diesem restlichen Bereich verbunden, so dass sie sich relativ zu diesem Bereich bewegen, insbesondere in Richtung zum Innenraum hin eindrücken lässt. Diese Richtung verläuft vorzugsweise parallel zur Längsrichtung des Stössels 32 und senkrecht zur Fläche des Deckels 33.

Vorzugsweise ist die Wandstärke der gesamten äusseren Wand 31 verdünnt im Vergleich zum restlichen, sie umgebenden Bereich des Gehäuseteils 1 ausgebildet. Vorzugsweise ist sie um ein Vielfaches dünner als dieser restliche Bereich. Es lässt sich jedoch auch lediglich das Scharnier bzw. der Übergangsbereich dünner und somit weicher ausbilden. Es sollte in diesem Fall jedoch durch entsprechende Form-und/oder Materialwahl gewährleistet sein, dass die äussere Wand 31 beim Eindrücken nicht so beschädigt wird, dass sie die Kammer 3 bzw. den Sekretraum 4 nicht mehr dicht verschliesst.

Die äussere Wand 31 ist hier nach aussen gewölbt ausgebildet, wobei ihre äusserste Erhebung mit der restlichen Wand des ersten Gehäuseteils 1 fluchtet. Diese Erhebung kann jedoch auch weiter nach aussen vorstehen oder vertieft in der restlichen Wand angeordnet sein.

Der Deckel 33 ist vorzugsweise flach ausgebildet. Vorzugsweise lässt er sich formschlüssig und vorzugsweise auch durch geeignete Bemassung (Untermass) kraftschlüssig auf den Mantel 30 aufsetzen, wobei ein umlaufender, radial vorstehender Flansch 332 als Anschlag dient. Ein dichter Verschluss ist erwünscht, aber nicht zwingend notwendig. Der Deckel 33 weist vorzugsweise radial verlaufende Rippen 330 auf, wie dies in den Figuren 1, 3 und 6 dargestellt ist.

Die Kammer 3 kann, wie im hier dargestellten Beispiel, einen einzigen Raum aufweisen oder in mehrere Räume unterteilt sein. Des Weiteren kann mehr als ein Stössel 32 vorhanden sein. Vorzugsweise ist die Kammer 3 in einem Bereich entfernt vom Drainage- und/oder Vakuumanschluss 10, 11 angeordnet. In diesem Beispiel ist sie im unteren Bereich des Behälters angeordnet.

Diese Kammer 3 dient der Aufnahme eines Verfestigungsmittels 4. In Figur 1 ist das Verfestigungsmittel 4 zylinderförmig dargestellt. Dies ist lediglich eine schematische Darstellung. Üblicherweise ist das Verfestigungsmittel ein Pulver oder ein Gel. Es kann bei Kontakt bzw. bei Vermischung mit der abgesaugten Körperflüssigkeit diese gelieren, verdicken, aus dieser Flocken bilden oder diese auf andere bekannte Arten verfestigen. Insbesondere wird ein Geliermittel verwendet, welches die Körperflüssigkeit koaguliert. Derartige Verfestigungsmittel sind im Stand der Technik hinlänglich bekannt und einige, die Liste der möglichen Mittel nicht abschliessende Beispiele wurden eingangs erwähnt.

Die oben beschriebene Vorrichtung lässt sich nun erfindungsgemäss wie folgt einsetzen:
Beim Transport und der Lagerung des Fluidsammelbehälters, d.h. vor dessen Gebrauch, befindet sich das Verfestigungsmittel 4 in der geschlossenen Kammer 3. Bei Gebrauch lässt sich die Kammer 3 durch äussere Krafteinwirkung öffnen und das Verfestigungsmittel 4 gelangt in die Sekretkammer 5. Dies kann nun kurz vor oder während der Absaugung der Körperflüssigkeit erfolgen. Vorzugsweise wird dies jedoch nach Beendigung der Absaugung durchgeführt. Je nach Art der Befestigung des Behälters kann dies vor oder nach seiner Entfernung von der Absaugvorrichtung erfolgen.

Die Öffnung der Kammer 3 erfolgt, indem mit der Hand oder einem geeigneten Gegenstand auf die äussere Wand 31 gedrückt wird, ohne diese jedoch zu zerstören. Die auf die Wand 31 ausgeübte Kraft überträgt sich auf den Stössel 32, welcher nun auf den Deckel 33 drückt und diesen vom Mantel 30 wegschiebt. Die Kammer 3 ist geöffnet und das Verfestigungsmittel 4 kann in den Innenraum gelangen. Dabei bleiben die Kammer 3 und somit auch der Sekretraum 5 nach wie vor nach aussen zur Umgebung hin luft- und flüssigkeitsdicht verschlossen.

Die Radialrippen 330 verteilen den Druck auf den Deckel 33 gleichmässig auf den Rand des Deckels 33, so dass dieser in diesem Beispiel ganz vom Mantel wegfällt. Durch geeignete Wahl der Form des Deckels bzw. der Lage und der Anzahl der Stössel kann der Deckel auch nur teilweise entfernt werden. Insbesondere kann er einseitig am Mantel befestigt sein. Er kann beispielsweise über ein Scharnier am Mantel einstückig angespritzt sein.

Der Deckel kann auch lediglich mit Löchern versehen werden. Hierzu kann er bereits Sollbruchstellen aufweisen oder er kann durchlöchert werden. Hierzu kann der Stössel mit einer oder mehreren Spitzen versehen sein. Des Weiteren kann anstelle des Deckels auch der Mantel mit Sollbruchstellen versehen sein bzw. durchlöchert werden, falls die äussere Wand mit einem entsprechend geformten Kraftübertragungsmittel verbunden ist.

In den oben beschriebenen Figuren ist lediglich ein bevorzugtes Beispiel eines erfindungsgemässen Fluidsammelbehälters dargestellt, wie er sich beispielsweise in einer medizinischen Drainagevorrichtung gemäss WO 2007/128156 einsetzen lässt. Die erfindungsgemässe Lehre ist jedoch nicht auf derartige starre Fluidsammelbehälter beschränkt. Insbesondere kann der Drainagebehälter auch durch einen flexiblen Beutel gebildet sein. Ist der Beutel mit einem starren Deckel zum Verschliessen eines starren Aussenbehälters verbunden, so kann die Kammer am Deckel angebracht oder angeformt sein. Ist kein starrer Deckel vorhanden, so kann die Kammer auch am Beutel selber angeordnet sein, wobei dann die äussere Wand der Kammer vorzugsweise durch eine zusätzliche, bei Bedarf entfernbare Abdeckung gegen ein ungewolltes Eindrücken geschützt ist.

Der erfindungsgemässe Fluidsammelbehälter ist kostengünstig herstellbar und verhindert eine Beschädigung der Kammer des Verfestigungsmittels vor dessen Gebrauch. Des Weiteren lässt sich die Kammer auf einfache und sichere Art und Weise bei Bedarf öffnen.

### Bezugszeichenliste

- 1: erstes Behälterteil
- 10: Vakuumanschluss
- 11: Drainageanschluss
- 12: Verschlussteil für Drainageanschluss
- 13: Überdruckventil
- 14: Befestigungszapfen
- 15: Ausnehmung für Verriegelungstaste
- 16: Rippe
- 16': Rippe

- 2: zweites Behälterteil

- 3: Kammer für Verfestigungsmittel
- 30: Mantel
- 31: Membran
- 32: Stössel
- 33: Deckel
- 330: Radialrippen
- 331: Aufnahme
- 332: Flansch

- 4: Verfestigungsmittel

- 5: Sekretkammer

- 6: Füllstandskala

## Patentansprüche

1. Fluidsammelbehälter zur Aufnahme eines abgesaugten Fluids, wobei der Fluidsammelbehälter eine geschlossene Kammer (3) für ein Verfestigungsmittel (4) aufweist, wobei sich die Kammer (3) durch äussere Krafteinwirkung zu einem Innenraum (5) des Fluidsammelbehälters hin öffnen lässt, wobei die Kammer (3) in den Innenraum (5) des Fluidsammelbehälters mindestens teilweise hinein ragt und wobei die Kammer (3) behälteraussenseitig mit einer nach innen drückbaren äusseren Wand (31) verschlossen ist, wobei sich die Kammer (3) durch das Eindrücken der äusseren Wand (31) zum Innenraum (5) des Fluidsammelbehälters hin öffnet, wobei der Innenraum (5) in diesem Bereich weiterhin nach aussen mit der äusseren Wand (31) verschlossen ist, wobei der Fluidsammelbehälter ein erstes und ein zweites Gehäuseteil (1, 2) aufweist, welche gemeinsam den Innenraum (5) des Fluidsammelbehälters bilden, **dadurch gekennzeichnet, dass** die äussere Wand (31) einstückig mit einer sie umgebenden Wand des ersten Gehäuseteils (1) ausgebildet ist.

2. Fluidsammelbehälter nach Anspruch 1, wobei die äussere Wand (31) flexibel ausgestaltet ist.

3. Fluidsammelbehälter nach einem der Ansprüche 1 oder 2, wobei in der Kammer (3) mindestens ein Übertragungsmittel (32) vorhanden ist, welches die Bewegung der äusseren Wand (31) auf mindestens eine innen liegende Wand (33) der Kammer (3) überträgt, um die Kammer (3) zu öffnen.

4. Fluidsammelbehälter nach Anspruch 3, wobei das mindestens eine Übertragungsmittel ein Stössel (32) ist, welcher auf einen der äusseren Wand (31) gegenüberliegenden, mindestens teilweise entfernbaren Deckel (33) der Kammer (3) wirkt.

5. Fluidsammelbehälter nach Anspruch 4, wobei der mindestens eine Stössel (32) mit der äusseren Wand (31) oder mit dem Deckel (33) verbunden ist, vorzugsweise einstückig mit der Wand (31) bzw. dem Deckel (33) ausgebildet ist.

6. Fluidsammelbehälter nach einem der Ansprüche 1 bis 5, wobei die bewegbare Wand (31) in einer Aussenwand des Sammelbehälters angeordnet ist.

7. Fluidsammelbehälter nach einem der Ansprüche 1 bis 6, wobei beide Gehäuseteile (1, 2) für sich einstückig ausgebildet sind.

8. Fluidsammelbehälter nach einem der Ansprüche 1 bis 7, wobei die Kammer (3) einen in den Innenraum (5) des Fluidsammelbehälters ragenden Mantel (30) aufweist, welcher einstückig mit der umgebenden Wand des Fluidsammelbehälters ausgebildet ist.

9. Fluidsammelbehälter nach einem der Ansprüche 1 bis 8, wobei er inkl. der Kammer (3) aus Kunststoff gefertigt ist.

10. Fluidsammelbehälter nach einem der Ansprüche 1 bis 9, wobei die Kammer (3) zylinderförmig und die äussere Wand (31) kreisförmig ausgebildet ist.

11. Fluidsammelbehälter nach einem der Ansprüche 1 bis 10 wobei der Innenraum eine Sekretkammer (5) zur Aufnahme des abgesaugten Fluids ist.

## Claims

1. A fluid-collecting container for receiving an aspirated fluid, wherein the fluid-collecting container has a closed chamber (3) for a solidifying agent (4), wherein the chamber (3) can be opened toward an internal space (5) in the fluid-collecting container by application of external force, wherein the chamber (3) protrudes at least partially into the internal space (5) in the fluid-collecting container, and wherein the chamber (3) is closed on the outer face of the container by an outer wall (31) that can be pressed inward, wherein the chamber (3) opens toward the internal space (5) in the fluid-collecting container when the outer wall (31) is pressed in, wherein the internal space (5) in this area remains closed off from the outside by the outer wall (31), wherein the fluid-collecting container has a first and a second housing part (1, 2) which together form the internal space (5) in the fluid-collecting container, **characterized in that** the outer wall (31) is formed in one piece with a surrounding wall of the first housing part (1).

2. The fluid-collecting container as claimed in claim 1, wherein the outer wall (31) is flexible.

3. The fluid-collecting container as claimed in either of claims 1 and 2, wherein at least one transmission member (32) is present in the chamber (3) and transmits the movement of the outer wall (31) to at least one inner wall (33) of the chamber (3) in order to open the chamber (3).

4. The fluid-collecting container as claimed in claim 3, wherein the at least one transmission member is a ram (32) acting on a lid (33) of the chamber (3), which lid (33) lies opposite the outer wall (31) and is at least partially removable.

5. The fluid-collecting container as claimed in claim 4, wherein the at least one ram (32) is connected to the outer wall (31) or to the lid (33), preferably being formed in one piece with the wall (31) or the lid (33).

6. The fluid-collecting container as claimed in one of claims 1 through 5, wherein the movable wall (31) is arranged in an outside wall of the collecting container.

7. The fluid-collecting container as claimed in one of claims 1 through 6, wherein each of the two housing parts (1, 2) is designed in one piece.

8. The fluid-collecting container as claimed in one of claims 1 through 7, wherein the chamber (3) has a jacket (30) which protrudes into the internal space (5) in the fluid-collecting container and which is formed in one piece with the surrounding wall of the fluid-collecting container.

9. The fluid-collecting container as claimed in one of claims 1 through 8, wherein it is made of plastic, including the chamber (3).

10. The fluid-collecting container as claimed in one of claims 1 through 9, wherein the chamber (3) is cylindrical and the outer wall (31) is circular.

11. The fluid-collecting container as claimed in one of claims 1 through 10, wherein the internal space is a secretion chamber (5) for receiving the aspirated fluid.

## Revendications

1. Un réservoir de collecte de fluide pour la réception d'un fluide aspiré, où le réservoir de collecte de fluide présente une chambre fermée (3) pour un agent solidifiant (4), où la chambre (3) peut être ouverte par application d'une force extérieure vers un espace intérieur (5) du réservoir de collecte de fluide, où la chambre (3) s'étend au moins partiellement dans l'espace intérieur (5) du réservoir de collecte de fluide et où la chambre (3) est fermée sur la face extérieure du boîtier par une paroi extérieure (31) qui peut être enfoncée vers l'intérieur, où la chambre (3) s'ouvre en enfonçant la paroi extérieure (31) vers l'espace intérieur (5) du réservoir de collecte de fluide, où l'espace intérieur (5) est en outre fermé vis-à-vis de l'extérieur par la paroi extérieure (31) dans ce domaine, où le réservoir de collecte de fluide présente une première et une seconde partie de boîtier (1, 2) lesquelles forment conjointement l'espace intérieur (5) du réservoir de collecte de fluide, **caractérisé en ce que** la paroi extérieure (31) forme une seule pièce avec une paroi de la première partie de boîtier (1) qui l'entoure.

2. Le réservoir de collecte de fluide selon la revendication 1, où la paroi extérieure (31) est formée de manière flexible.

3. Le réservoir de collecte de fluide selon une des revendications 1 ou 2, où il existe au moins un moyen de transmission (32) dans la chambre (3), qui transmet le mouvement de la paroi extérieure (31) sur au moins une paroi située à l'intérieur (33) de la chambre (3), pour ouvrir la chambre (3).

4. Le réservoir de collecte de fluide selon la revendication 3, où l'au moins un moyen de transmission est un poussoir (32), lequel agit sur un couvercle (33) de la chambre (3) situé en opposé à la paroi extérieure (31) au moins partiellement amovible.

5. Le réservoir de collecte de fluide selon la revendication 4, où l'au moins un poussoir (32) est connecté avec la paroi extérieure (31) ou avec le couvercle (33), préférablement forme une seule pièce avec la paroi (31), respectivement le couvercle (33).

6. Le réservoir de collecte de fluide selon une des revendications 1 à 5, où la paroi (31) mobile est disposée dans une paroi extérieure du réservoir de collecte.

7. Le réservoir de collecte de fluide selon une des revendications 1 à 6, où les deux parties de boîtier (1, 2) forment une seule pièce en elles-mêmes.

8. Le réservoir de collecte de fluide selon une des revendications 1 à 7, où la chambre (3) présente une gaine (30) s'étendant dans l'espace intérieur (5) du réservoir du collecte de fluide, laquelle forme une seule pièce avec la paroi entourante du réservoir du collecte de fluide.

9. Le réservoir de collecte de fluide selon une des revendications 1 à 8, où celui-ci est formé en matière plastique, y compris la chambre (3).

10. Le réservoir de collecte de fluide selon une des revendications 1 à 9, où la chambre (3) est formée de manière cylindrique, et la paroi extérieure (31) est formée de manière circulaire.

11. Le réservoir de collecte de fluide selon une des revendications 1 à 10, où l'espace intérieure est une chambre à sécrétions (5) pour la réception du fluide aspirée.
